# EUROPEAN PATENT APPLICATION

(11) **EP 4 650 384 A1**
(43) Date of publication of application: **19.11.2025**
(21) Application number: 25176895.8
(22) Date of filing: 16.05.2025
(51) Int. Cl.: C08G 73/02, A61K 48/00, C12N 15/88

(54) **MODIFIED CATIONIC POLYMER AND USE THEREOF**

(30) Priority: 17.05.2024 CN 202410616873
(71) Applicant: Yeasen Biotechnology (Shanghai) Co., Ltd., Shanghai 201318 (CN)
(72) Inventor: MING, Cong, Shanghai, 201318 (CN); TENG, Yigang, Shanghai, 201318 (CN); GUO, Shunfeng, Shanghai, 201318 (CN); LIU, Houlei, Shanghai, 201318 (CN)
(74) Representative: Comoglio, Elena

(57) **Abstract**

Provided is a modified cationic polymer and use thereof. The modified cationic polymer has a structure shown by Formula I or Formula II. The modified cationic polymer provided by the present application has small molecules grafted onto the cationic polymer, and thus the obtained modified cationic polymer has high transfection efficiency.

## Description

### TECHNICAL FIELD

The present application belongs to the field of gene transfection biotechnology, and particularly relates to a modified cationic polymer and use thereof.

### BACKGROUND

Cell transfection refers to the technology of deliberately introducing exogenous gene molecules such as DNA and RNA into cells. With the rapid development of cell biology and molecular biology and the in-depth study of gene and protein functions, transfection has become a regular experimental method in laboratory work. Transfection reagent is needed in transfection to transport vectors carrying the target gene into the cells. At present, cationic polymers is a class of transfection reagent commonly used; its advantage lies in that the main chain of the cationic polymers can provide a large number of positively charged groups. The cationic polymers with positive charges are capable of making DNA into particles or aggregates at physiological pH, and facilitating the DNA binding to cells and entering cells by endocytosis.

CN104419004A discloses a modified polyethyleneimine, a preparation method thereof, a gene transfection reagent and use thereof. The modified polyethyleneimine includes the main chain polyethyleneimine and ε-caprolactone grafted on the polyethyleneimine, wherein the ε-caprolactone is grafted by coordination ring-opening on the primary amine or secondary amine of the polyethyleneimine, and coordination ring-opening polymerization of the ε-caprolactone does not occur. CN104974343A discloses a modified polyethyleneimine and its use in preparing gene transfection vectors, wherein the modified polyethyleneimine has hydrophobic rosin acid or dehydroabietic acid grated on the polyethyleneimine.

In view of the low transfection efficiency of the existing cationic polymers, it is urgent to design a modified cationic polymer with high transfection efficiency.

### SUMMARY

Aiming at the defects of the prior art, the present application is to provide a modified cationic polymer and use thereof. By grafting small molecules onto the cationic polymer chains, the obtained modified cationic polymer has high transfection efficiency.

To achieve the object, technical solutions below are adopted in the present application.

In a first aspect, the present application provides a modified cationic polymer having a structure shown by Formula I or Formula II:

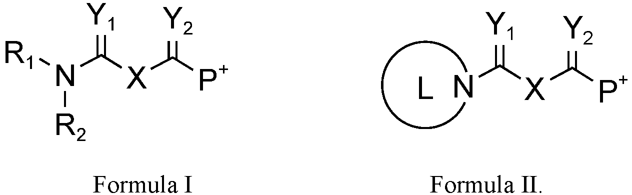

In Formula I and Formula II, R₁ and R₂ are each independently selected from any one of hydrogen, substituted or unsubstituted amino, substituted or unsubstituted C1-C20 linear or branched alkyl, substituted or unsubstituted C3-C30 cycloalkyl, C2-C30 heterocycloalkyl, substituted or unsubstituted C1-C20 alkoxy, substituted or unsubstituted C6-C30 aryl, and substituted or unsubstituted C3-C30 heteroaryl; one or at least two nonadjacent -CH₂- in the C1-C20 linear or branched alkyl can each independently be substituted with -O-.

In the present application, the expression "one or at least two nonadjacent -CH₂- in the C1-C20 linear or branched alkyl can each independently be substituted with -O-" means that, for example, one -CH₂- in -CH₂-CH₂-CH₃ is substituted with -O- to give -O-CH₂-CH₃ or -CH₂-O-CH₃.

X is selected from any one of a single bond, substituted or unsubstituted C1-C10 linear or branched alkylene, C2-C10 linear or branched alkenylene, C2-C10 linear or branched alkynylene, C3-C10 cycloalkylene, C3-C10 heterocycloalkylene, and C3-C10 unsaturated cycloalkylene; in the C1-C10 linear or branched alkylene, one or at least two nonadjacent -CH₂- can each independently be substituted with -O- or -S-, and one or at least two nonadjacent -CH- can each independently be substituted with -N-; when X is a single bond, C atoms which are joined with Y₁ and Y₂ respectively are directly connected by the single bond.

Y₁ and Y₂ are each independently selected from O or S.

The ring L is selected from any one of a substituted or unsubstituted C3-C10 N-containing heteroalicyclic ring, and a substituted or unsubstituted C3-C12 N-containing heteroaromatic ring; a heteroatom in the C3-C10 N-containing heteroalicyclic ring and the C3-C12 N-containing heteroaromatic ring is selected from at least one of N, O or S.

P⁺ represents a cationic polymer; the cationic polymer includes any one of cationic polymers containing primary amine and/or secondary amine; in addition to the primary amine and/or secondary amine, the cationic polymers may further contain tertiary amine.

Substituents for substitution in R₁, R₂, X, and the ring L are each independently selected from at least one of unsubstituted or Z₁-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino; M₁ is selected from any one of C1-C10 linear or branched alkyl; Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

For the modified cationic polymer provided by the present application, the cationic polymer chains are grafted with small molecules. Because amide bonds or thioamide bonds contained in the modified cationic polymer can form hydrogen bonds with heteroatoms on nucleic acids, the nucleic acid molecules are further compressed, the resulting firmer complexes can be more easily taken into cells through endocytosis, and thereby the modified cationic polymer has higher transfection efficiency.

The modified cationic polymer provided by the present application has a structure shown by Formula I or Formula II, or a tautomer, mesomer, racemate, enantiomer, diastereomer or a mixture thereof, or an acceptable salt thereof, and an acceptable excipient, a buffer, a cell culture medium or a transfection medium, etc.

In the present application, the C1-C20 may each independently be C1, C2, C3, C4, C5, C6, C7, C8, C9, C10, C11, C12, C13, C14, C15, C16, C17, C18, C19, etc.

The C3-C30 may each independently be C3, C4, C5, C6, C7, C8, C9, C10, C11, C12, C13, C14, C15, C16, C17, C18, C19, C20, C22, C24, C26, C28, C30, etc.

The C2-C30 may each independently be C2, C3, C4, C5, C6, C7, C8, C9, C10, C11, C12, C13, C14, C15, C16, C17, C18, C19, C20, C22, C24, C26, C28, C30, etc.

The C6-C30 may each independently be C6, C8, C9, C10, C12, C13, C14, C15, C16, C18, C20, C22, C24, C26, C28, C30, etc.

The C1-C10 may each independently be C1, C2, C3, C4, C5, C6, C7, C8, C9, C10, etc.

The C2-C10 may each independently be C2, C3, C4, C5, C6, C7, C8, C9, C10, etc.

The C3-C10 may each independently be C3, C4, C5, C6, C7, C8, C9, C10, etc.

The C3-C12 may each independently be C3, C4, C5, C6, C7, C8, C9, C10, C11, C12, etc.

The C6-C12 may each independently be C6, C7, C8, C9, C10, C11, C12, etc.

The following is preferred technical solutions of the present application, but not limitation to the technical solutions provided by the present application. The objects and beneficial effects of the present application can be better achieved and realized by the following preferred technical solutions.

In the present application, the halogen includes F, Cl, Br or I.

The C1-C20 (such as C1, C2, C3, C4, C5, C6, C7, C8, C9, C10, C11, C12, C13, C14, C15, C16, C17, C18, C19, etc.) linear or branched alkyl (preferably, C1-C10 linear or branched alkyl) illustratively includes, but is not limited to, methyl, ethyl, n-propyl, isopropyl, n-butyl, isobutyl, tert-butyl, n-pentyl, isopentyl, neopentyl, tert-pentyl, n-hexyl, n-octyl, n-heptyl, n-nonyl, n-decyl, etc.

The C3-C30 cycloalkyl (preferably, C3-C10 cycloalkyl) illustratively includes, but is not limited to, cyclopropyl, cyclobutyl, cyclopentyl, cyclohexyl, adamantyl, etc.

The C2-C30 heterocycloalkyl (preferably, C2-C10 heterocycloalkyl) illustratively includes, but is not limited to, morpholinyl, piperazinyl, piperidinyl, tetrahydropyrrolyl, etc.

The C1-C20 alkoxy is a monovalent group formed by connecting O with the linear or branched alkyl listed above.

The C6-C30 aryl (preferably, C6-C20 aryl) illustratively includes, but is not limited to, phenyl, biphenyl, terphenyl, naphthyl, anthryl, phenanthryl, indenyl, fluorenyl and derivatives thereof (9,9-dimethylfluorenyl, 9,9-diethylfluorenyl, 9,9-diphenylfluorenyl, 9, 9-dinaphthylfluorenyl, spirobifluorenyl, benzofluorenyl, etc), fluoranthene, triphenylene, pyrene, perylene, chrysene, tetracene, benzophenanthryl, etc.

The C3-C30 heteroaryl (preferably, C3-C20 heteroaryl) illustratively includes, but is not limited to, furyl, thienyl, pyrrolyl, pyridinyl, pyrazinyl, pyridazinyl, pyrimidinyl, triazinyl, quinolinyl, isoquinolinyl, quinazolinyl, quinoxalinyl, cinnoline, phenanthroline, imidazolyl, thiazolyl, oxazolyl, benzoimidazolyl, benzothiazolinyl, benzoxazolyl, benzofuranyl, benzothienyl, indolyl, dibenzofuranyl, dibenzothienyl, carbazolyl and derivatives thereof (N-phenylcarbazolyl, N-naphthylcarbazolyl, benzocarbazolyl, dibenzocarbazolyl, indolocarbazolyl, azacarbazolyl, etc.), phenothiazinyl, phenoxazinyl, hydrogenated acridinyl, etc.

The C2-C10 linear or branched alkenylene illustratively includes, but is not limited to, vinylidene, propenylidene, butenylidene, etc.

The C3-C10 unsaturated cycloalkylene illustratively includes, but is not limited to, cyclopentenylidene, cyclohexenylidene, cyclohexadienylidene, etc.

In the present application, the "substituted or unsubstituted" group may have one substituent or a plurality of substituents; when there are a plurality of (at least two) substituents, the plurality of (at least two) substituents are the same or different; the substituent may be joined to any position applicable to being joined. The same expression hereinafter refers to the same meaning.

As a preferred technical solution, R₁ and R₂ are each independently selected from any one of hydrogen, substituted or unsubstituted amino, substituted or unsubstituted C1-C10 (such as C1, C2, C3, C4, C5, C6, C7, C8, C9 or C10) linear or branched alkyl, substituted or unsubstituted C3-C10 (such as C3, C4, C5, C6, C7, C8, C9 or C10) cycloalkyl, C2-C10 (such as C2, C3, C4, C5, C6, C7, C8, C9, or C10) heterocycloalkyl, substituted or unsubstituted C1-C10 (such as C1, C2, C3, C4, C5, C6, C7, C8, C9 or C10) alkoxy, substituted or unsubstituted C6-C20 (such as C6, C8, C9, C10, C12, C13, C14, C15, C16, C18 or C20) aryl, and substituted or unsubstituted C3-C20 (such as C3, C4, C5, C6, C8, C9, C10, C12, C13, C14, C15, C16, C18 or C20) heteroaryl; one or at least two nonadjacent -CH₂- in the C1-C10 linear or branched alkyl can each independently be substituted with -O-; a substituent for substitution is selected from at least one of unsubstituted or Zi-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino; M₁ is selected from any one of C1-C10 linear or branched alkyl; Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

Preferably, one of R₁ and R₂ is hydrogen, and the other one is selected from any one of substituted or unsubstituted amino, substituted or unsubstituted C1-C10 (such as C1, C2, C3, C4, C5, C6, C7, C8, C9 or C10) linear or branched alkyl, C3-C10 (such as C3, C4, C5, C6, C7, C8, C9 or C10) cycloalkyl, substituted or unsubstituted C6-C20 (such as C6, C8, C9, C10, C12, C13, C14, C15, C16, C18 or C20) aryl, and substituted or unsubstituted C3-C20 (such as C3, C4, C5, C6, C8, C9, C10, C12, C13, C14, C15, C16, C18 or C20) heteroaryl; one or at least two nonadjacent -CH₂- in the C1-C10 linear or branched alkyl can each independently be substituted with -O-.

A substituent for substitution is selected from at least one of unsubstituted or Z₁-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino; M₁ is selected from any one of C1-C10 linear or branched alkyl; Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

Preferably, one of R₁ and R₂ is hydrogen, and the other one is selected from any one of substituted or unsubstituted C1-C5 linear alkyl, substituted or unsubstituted amino, CH₃O(CH₂)₂O(CH₂)₂-, CH₃O(CH₂)₂O(CH₂)₂O(CH₂)₂-, and the following groups in substituted or unsubstituted form: 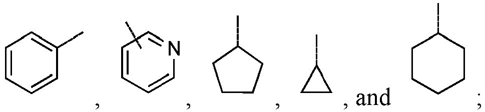 wherein the dotted line represents a linkage site for the group; a substituent for substitution is selected from at least one of unsubstituted or Z₁-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino; M₁ is selected from any one of C1-C10 linear or branched alkyl; Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

Preferably, X is selected from any one of a single bond, C1-C5 (such as C1, C2, C3, C4, or C5) linear or branched alkylene, C2-C5 (such as C2, C3, C4, or C5) linear or branched alkenylene, C2-C5 (such as C2, C3, C4, or C5) linear or branched alkynylene, C3-C6 (such as C3, C4, C5, or C6) cycloalkylene, C3-C6 (such as C3, C4, C5, or C6) heterocycloalkylene, and C3-C6 (such as C3, C4, C5, or C6) unsaturated cycloalkylene; in the C1-C5 (such as C1, C2, C3, C4, or C5) linear or branched alkylene, one or at least two nonadjacent -CH₂- can each independently be substituted with -O- or -S-, and one or at least two nonadjacent -CH- can each independently be substituted with -N-.

Preferably, X is selected from any one of ethylidene, propylidene, butylidene, vinylidene or propenylidene.

Preferably, the ring L is selected from any one of a C3-C6 (such as C3, C4, C5, or C6) N-containing heteroalicyclic ring and a C3-C7 (such as C3, C4, C5, C6, or C7) N-containing heteroaromatic ring.

Preferably, the ring L is selected from any one of the following groups: 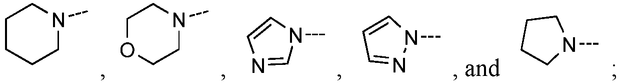 wherein the dotted line represents a linkage site for the group.

The cationic polymer represents a class of nucleic acid delivery agent. The cationic polymer in the present application is selected from: linear or branched polyethyleneimine (PEI), PEI dendrimer, polypropyleneimine (PPI), polybutyleneimine (PBI), polyamide (PAMAM), cationic cyclodextrin, polyalkylamine, polyhydroxyalkylamine, spermine, N-substituted polyallylamine, N-substituted chitosan, polylysine, polyvinylamine, poly(β-amino ester), hyperbranched poly(amino ester) (h-PAE), network poly(amino ester) (n-PAE), and poly(4-hydroxy-1-proline ester) (PHP-ester).

Preferably, the cationic polymer is linear or branched polyethylene imine (PEI). In some embodiments of the present application, the cationic polymer is linear PEI.

Preferably, the cationic polymers containing primary amine and/or secondary amine include any one of branched polyethyleneimine, linear polyethyleneimine, polyallylamine, polylysine or polyvinylamine, and further preferably linear polyethyleneimine.

Preferably, the cationic polymers containing primary amine and/or secondary amine have a weight average molecular mass of 5000-50000 (such as 5000, 10000, 15000, 20000, 25000, 30000, 35000, 40000, 45000, etc.), and further preferably 15000-40000.

Preferably, the modified cationic polymer has a grafting rate of 8-36%, which may be, for example, 8%, 9%, 10%, 11%, 12%, 14%, 16%, 18%, 20%, 22%, 24%, 26%, 28%, 30%, 32%, 34%, 36%, etc.

The grafting rate is calculated using the following formula: grafting rate = number of monomers grafted on the primary amine and/or secondary amine of the cationic polymer by side chains/number of total monomers present in the original cationic polymer × 100%; generally, NMR is used for the measurement.

Preferably, the modified cationic polymer is modified linear polyethyleneimine having any one of the following structures:

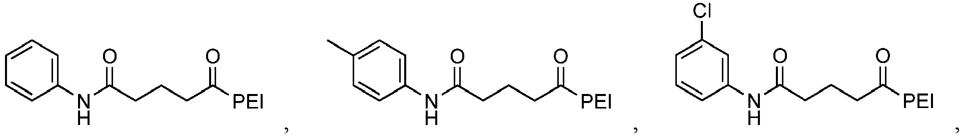

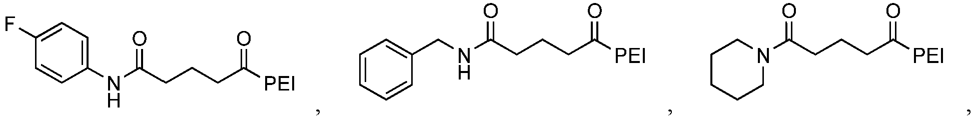

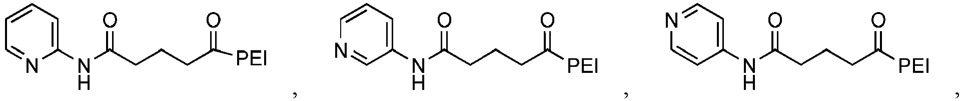

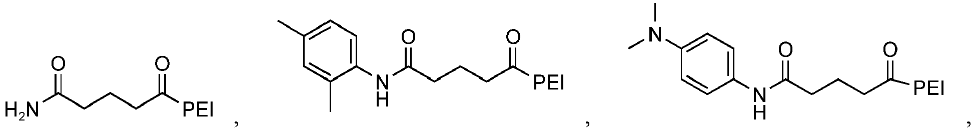

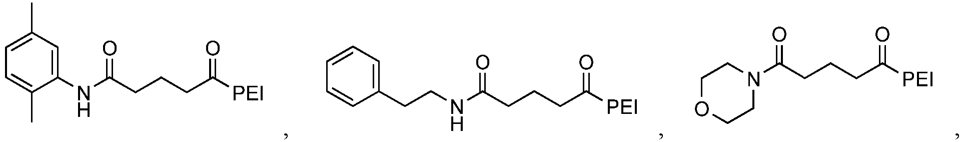

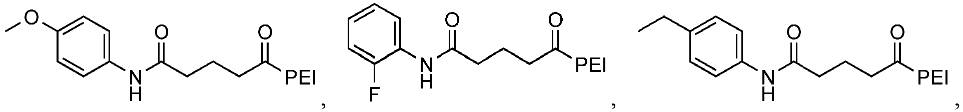

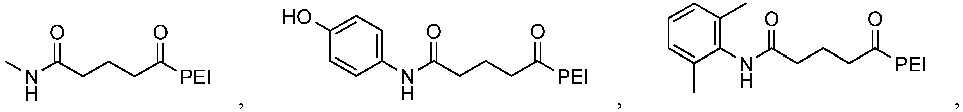

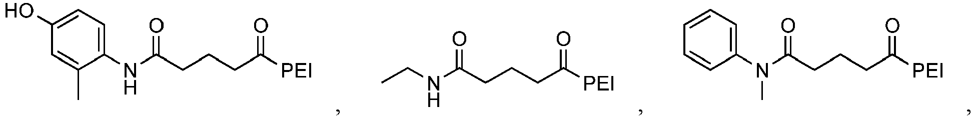

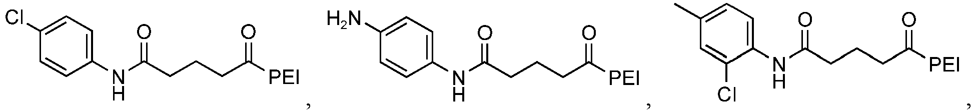

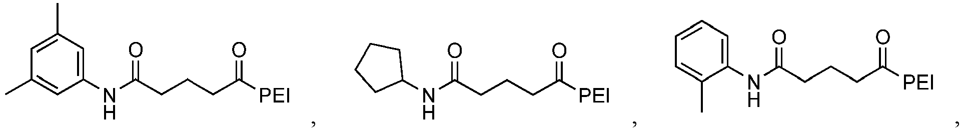

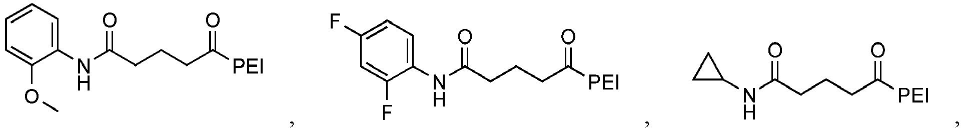

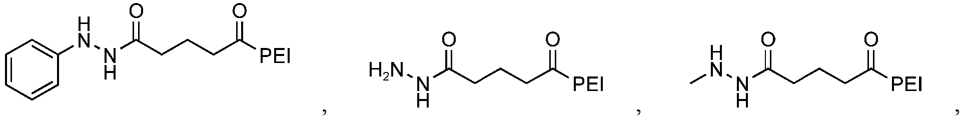

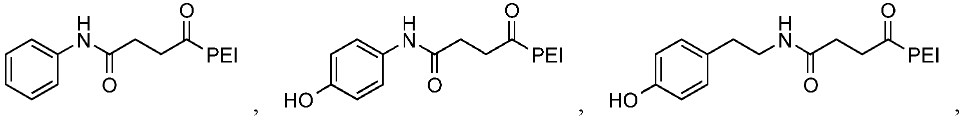

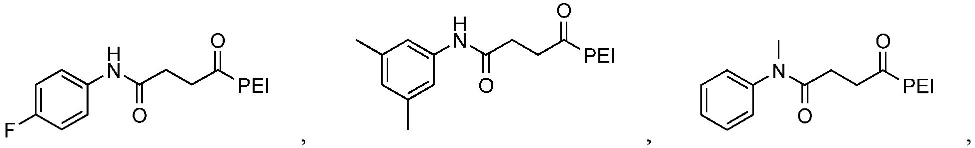

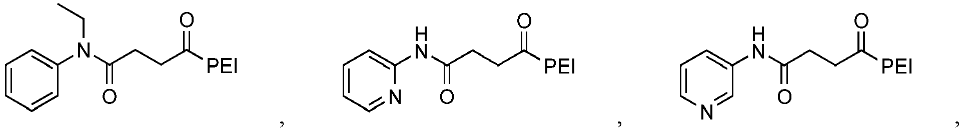

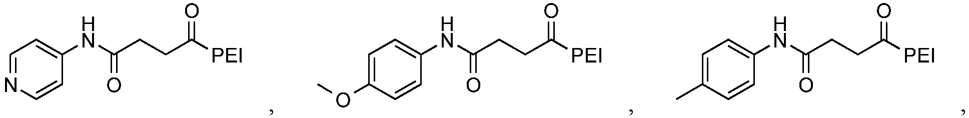

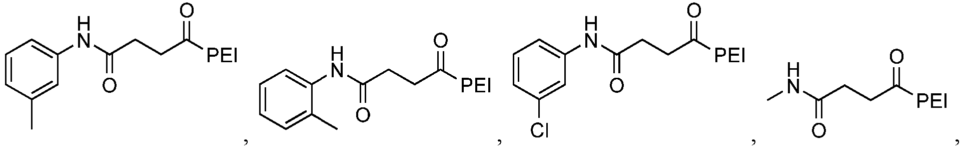

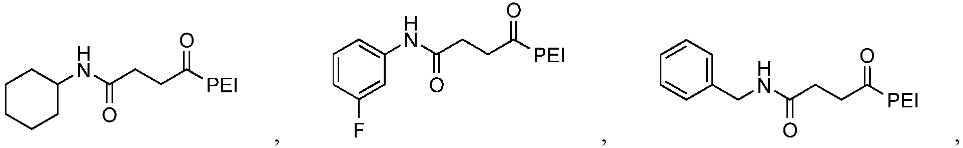

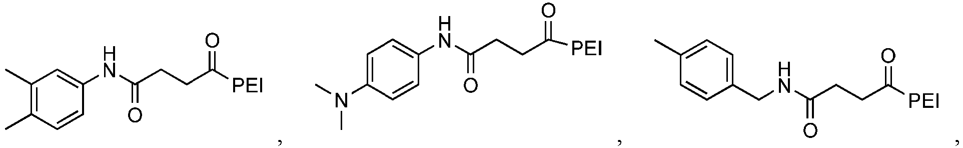

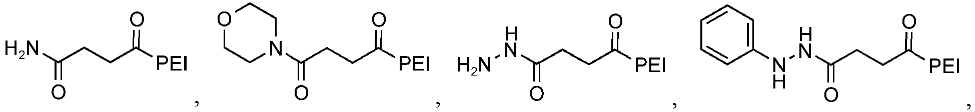

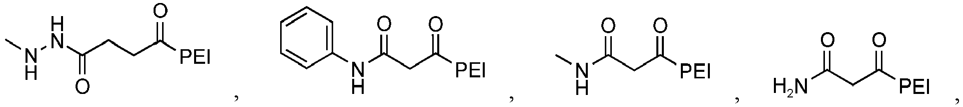

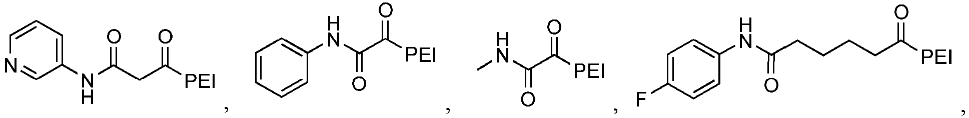

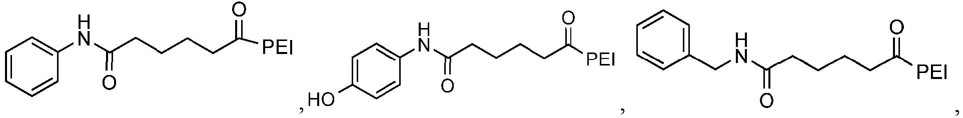

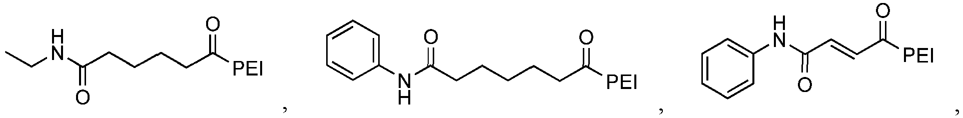

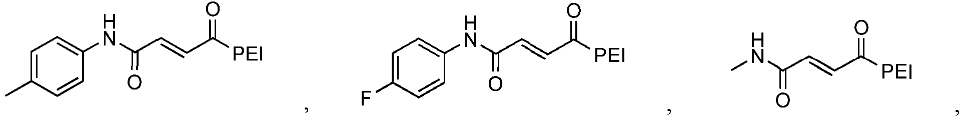

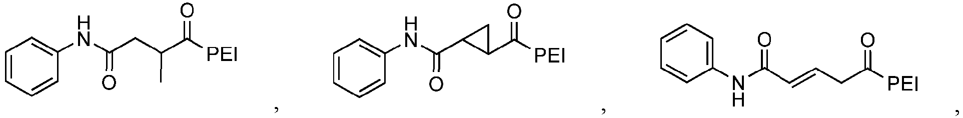

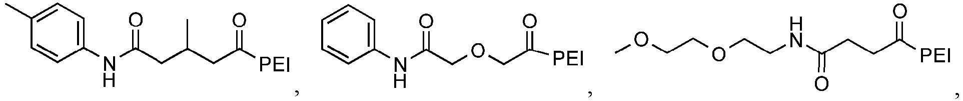

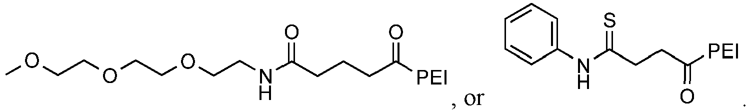

Preferably, the modified cationic polymer includes any one of the following modified linear polyethyleneimines 101-190:

| No. | Structure | Weight average molecular mass of linear polyethyleneimine | Grafting rate |
|---|---|---|---|
| 101 | 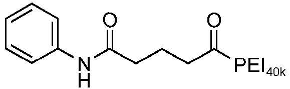 | 40K | 10% |
| 102 | 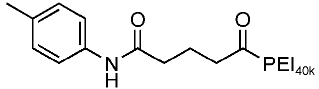 | 40K | 20% |
| 103 | 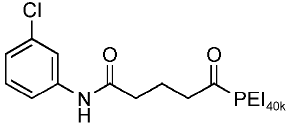 | 40K | 13% |
| 104 | 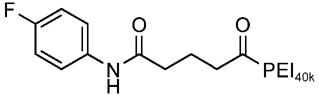 | 40K | 18% |
| 105 | 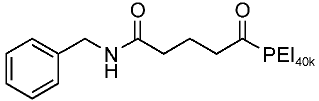 | 40K | 17% |
| 106 | 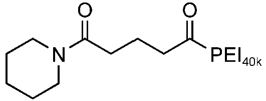 | 40K | 20% |
| 107 | 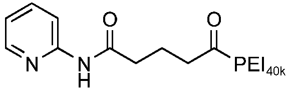 | 40K | 15% |
| 108 | 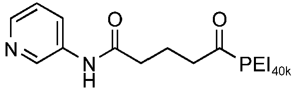 | 40K | 8% |
| 109 | 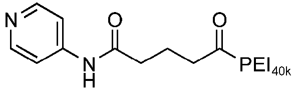 | 40K | 10% |
| 110 | 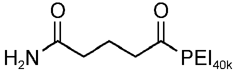 | 40K | 20% |
| 111 | 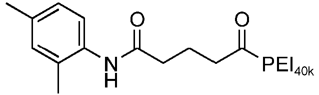 | 40K | 20% |
| 112 | 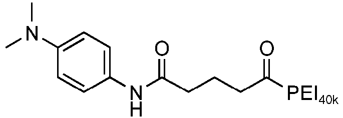 | 40K | 25% |
| 113 | 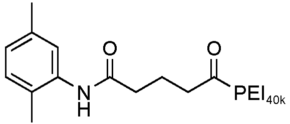 | 40K | 21% |
| 114 | 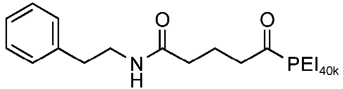 | 40K | 15% |
| 115 | 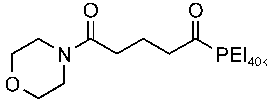 | 40K | 20% |
| 116 | 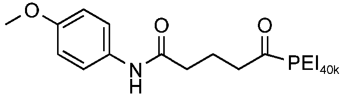 | 40K | 20% |
| 117 | 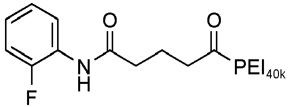 | 40K | 15% |
| 118 | 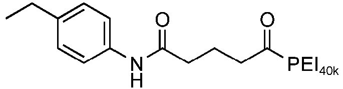 | 40K | 18% |
| 119 | 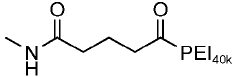 | 40K | 16% |
| 120 | 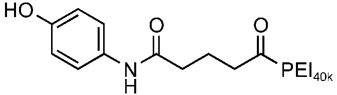 | 40K | 18% |
| 121 | 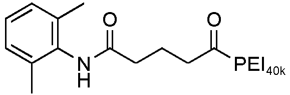 | 40K | 10% |
| 122 | 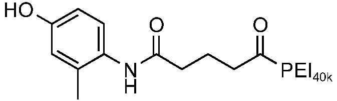 | 40K | 15% |
| 123 | 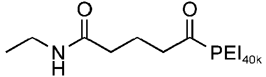 | 40K | 18% |
| 124 | 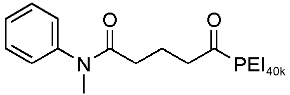 | 40K | 20% |
| 125 | 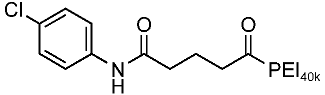 | 40K | 18% |
| 126 | 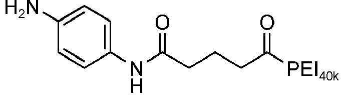 | 40K | 25% |
| 127 | 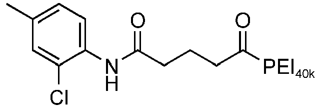 | 40K | 26% |
| 128 | 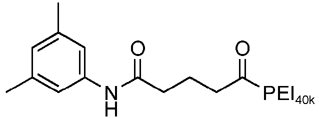 | 40K | 30% |
| 129 | 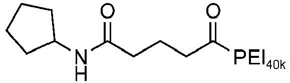 | 40K | 20% |
| 130 | 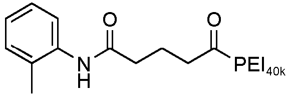 | 40K | 30% |
| 131 | 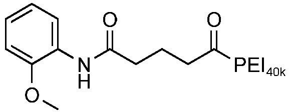 | 40K | 20% |
| 132 | 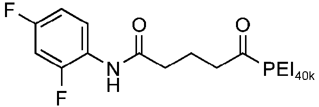 | 40K | 20% |
| 133 | 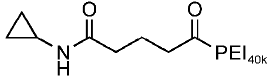 | 40K | 10% |
| 134 | 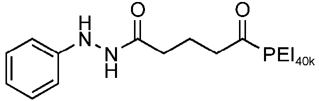 | 40K | 15% |
| 135 | 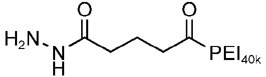 | 40K | 20% |
| 136 | 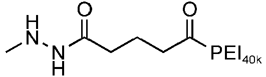 | 40K | 20% |
| 137 | 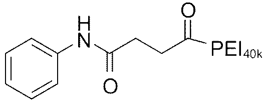 | 40K | 20% |
| 138 | 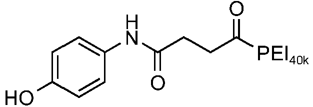 | 40K | 35% |
| 139 | 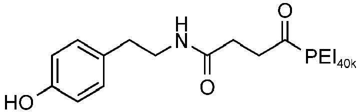 | 40K | 20% |
| 140 | 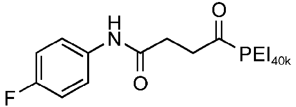 | 40K | 25% |
| 141 | 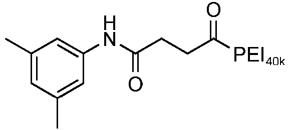 | 40K | 25% |
| 142 | 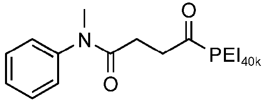 | 40K | 10% |
| 143 | 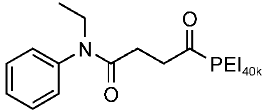 | 40K | 15% |
| 144 | 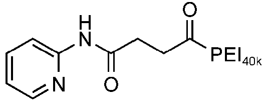 | 40K | 25% |
| 145 | 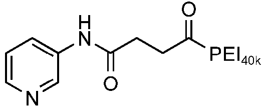 | 40K | 24% |
| 146 | 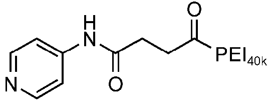 | 40K | 20% |
| 147 | 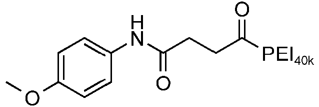 | 40K | 18% |
| 148 | 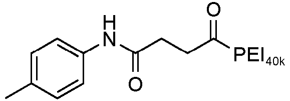 | 40K | 28% |
| 149 | 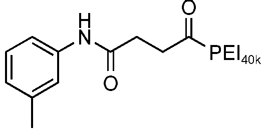 | 40K | 35% |
| 150 | 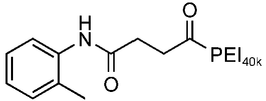 | 40K | 15% |
| 151 | 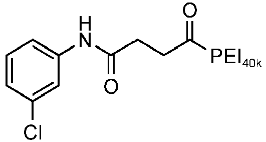 | 40K | 20% |
| 152 | 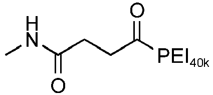 | 40K | 10% |
| 153 | 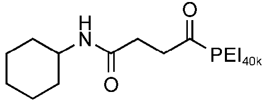 | 40K | 17% |
| 154 | 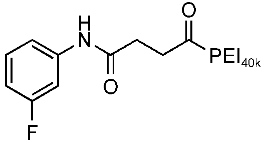 | 40K | 26% |
| 155 | 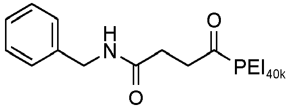 | 40K | 28% |
| 156 | 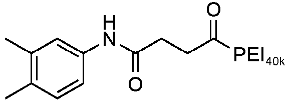 | 40K | 20% |
| 157 | 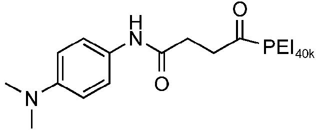 | 40K | 15% |
| 158 | 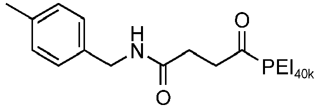 | 40K | 24% |
| 159 | 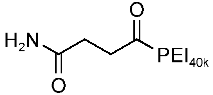 | 40K | 20% |
| 160 | 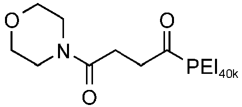 | 40K | 26% |
| 161 | 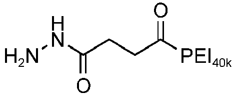 | 40K | 15% |
| 162 | 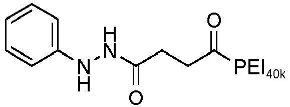 | 40K | 9% |
| 163 | 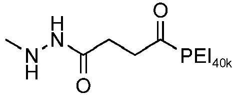 | 40K | 13% |
| 164 | 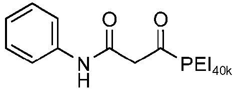 | 40K | 17% |
| 165 | 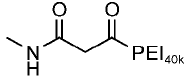 | 40K | 16% |
| 166 | 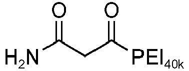 | 40K | 15% |
| 167 | 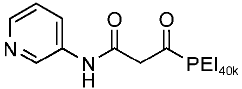 | 40K | 20% |
| 168 | 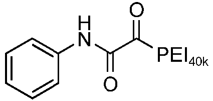 | 40K | 10% |
| 169 | 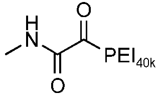 | 40K | 25% |
| 170 | 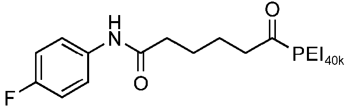 | 40K | 26% |
| 171 | 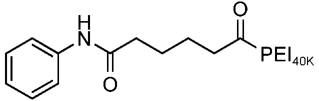 | 40K | 15% |
| 172 | 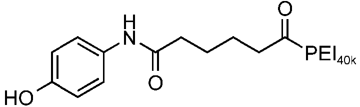 | 40K | 17% |
| 173 | 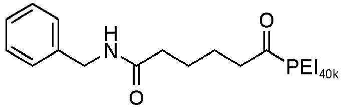 | 40K | 22% |
| 174 | 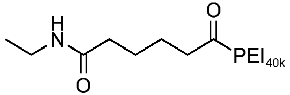 | 40K | 16% |
| 175 | 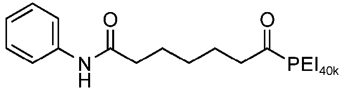 | 40K | 20% |
| 176 | 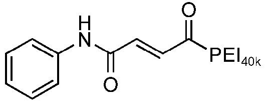 | 40K | 20% |
| 177 | 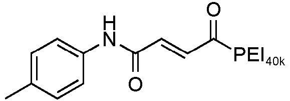 | 40K | 17% |
| 178 | 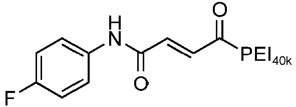 | 40K | 24% |
| 179 | 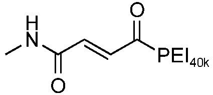 | 40K | 12% |
| 180 | 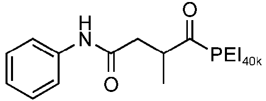 | 40K | 36% |
| 181 | 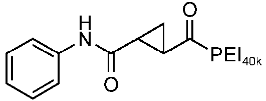 | 40K | 13% |
| 182 | 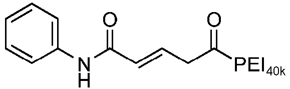 | 40K | 15% |
| 183 | 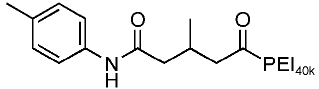 | 40K | 20% |
| 184 | 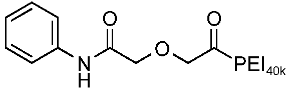 | 40K | 10% |
| 185 | 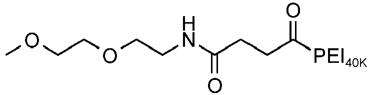 | 40K | 20% |
| 186 | 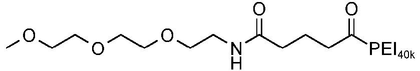 | 40K | 16% |
| 187 | 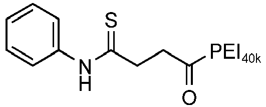 | 40K | 20% |
| 188 | 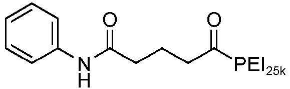 | 25K | 10% |
| 189 | 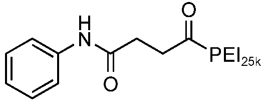 | 25K | 20% |
| 190 | 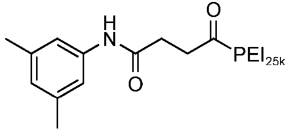 | 25K | 25%. |

Preferably, the modified cationic polymer has a structure shown by Formula I-1 or Formula II-1:

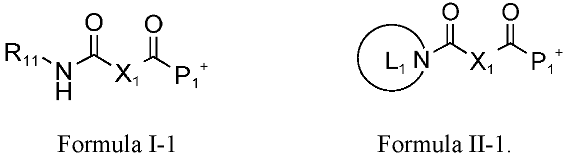

In Formula I-1 and Formula II-1, R₁₁ is selected from any one of phenyl-substituted C1-C5 linear alkyl, phenyl-substituted amino, substituted or unsubstituted C6-C12 aryl, substituted or unsubstituted C3-C12 heteroaryl, and C3-C10 cycloalkyl.

X₁ is selected from any one of a single bond and C1-C4 linear alkylene; one or at least two nonadjacent -CH₂- in the C1-C4 linear alkylene can each independently be substituted with -O-.

The ring L₁ is selected from any one of a substituted or unsubstituted C3-C10 N-containing heteroalicyclic ring, and a substituted or unsubstituted C3-C12 N-containing heteroaromatic ring.

P₁⁺ represents linear polyethyleneimine.

A substituent for substitution is selected from at least one of halogen, hydroxyl, and C1-C10 linear or branched alkyl.

Preferably, the R₁₁ is selected from any one of the following groups: 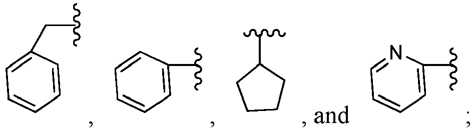 wherein 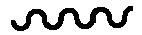 represents a linkage site for the group.

Preferably, the R₁₁ is selected from any one of C6-C12 aryl.

Preferably, the X₁ is selected from any one of C1-C4 linear alkylene.

Preferably, the modified cationic polymer includes any one of the following modified linear polyethyleneimines:

| Structure | Weight average molecular mass of linear polyethyleneimine | Grafting rate |
|---|---|---|
| 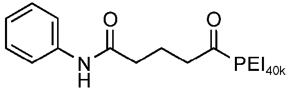 | 40K | 10% |
| 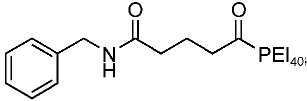 | 40K | 17% |
| 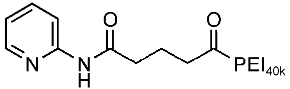 | 40K | 15% |
| 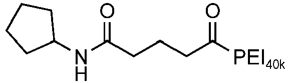 | 40K | 20% |
| 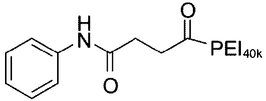 | 40K | 20% |
| 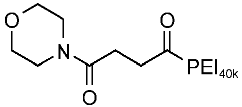 | 40K | 26% |
| 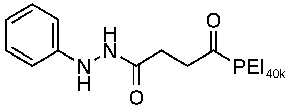 | 40K | 9% |
| 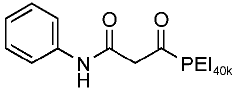 | 40K | 17% |
| 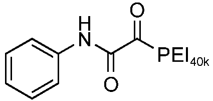 | 40K | 10% |
| 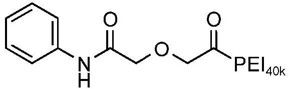 | 40K | 10%. |

Illustratively,, the modified cationic polymer having a structure shown by Formula I or Formula II provided by the present application can be prepared by the following synthetic route:

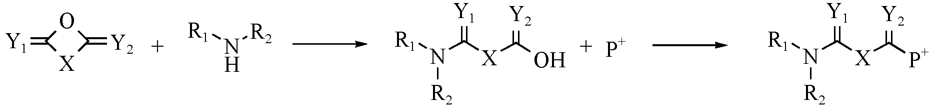

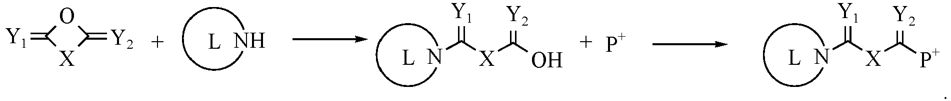

In the synthetic route, R₁, R₂, X, Y₁, Y₂, the ring L and P⁺ are defined in the same manner as the structure shown by Formula I or Formula II.

In a third aspect, the present application provides a tautomer, mesomer, racemate, enantiomer, diastereomer or acceptable salt of the modified cationic polymer as described in the first aspect.

In a fourth aspect, the present application provides a transfection reagent, and the transfection reagent includes any one or a combination of at least two of the modified cationic polymer as described in the first aspect, or the tautomer, mesomer, racemate, enantiomer, diastereomer or acceptable salt of the modified cationic polymer as described in the second aspect.

Compare with that prior art, the present application has the following beneficial effects:
For the modified cationic polymer provided by the present application, grafting small molecules onto the cationic polymer chains endows the cationic polymer with excellent transfection performance, and thus the 293F GFP positive rate of the modified cationic polymer can be maintained at 70% or more.

### BRIEF DESCRIPTION OF DRAWINGS

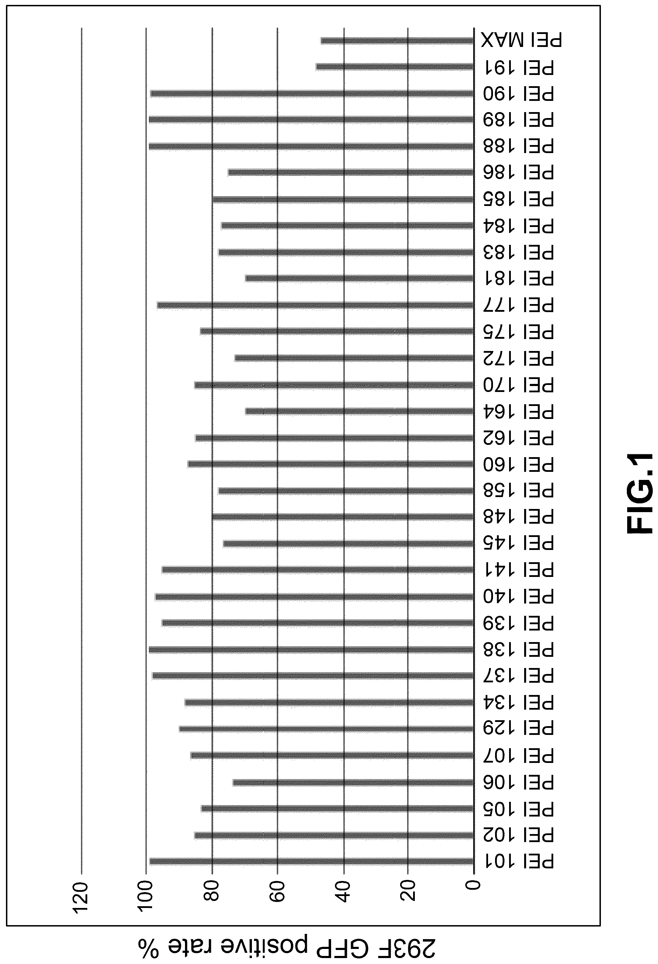

FIG. 1 shows comparison of 293F GFP positive rate of a modified linear polyethyleneimine provided by the present application.

### DETAILED DESCRIPTION

The technical solutions of the present application are further described below with reference to the accompanying drawing and specific embodiments. It should be apparent to those skilled in the art that the described embodiments are merely used for a better understanding of the present application and should not be construed as a specific limitation on the present application.

Sources of some components in the examples and comparative examples are as follows:
(1) linear polyethyleneimine: purchased from Yeasen Biotechnology (Shanghai) Co., Ltd., with a weight average molecular mass of 40 K;
(2) Intermediate S160: purchased from JW&Y PharmLab Co., Ltd.;
(3) Intermediate S164: purchased from JW&Y PharmLab Co., Ltd.;
(4) Intermediate S168: purchased from Tianjin Heowns Biochemical Technology Co., Ltd.;
(5) Intermediate S191: purchased from Shanghai Aladdin biochemical technology Co., Ltd.

### Example 1

A modified linear polyethyleneimine 137 (PEI 137) is provided, and a preparation method of PEI 137 includes the following steps: 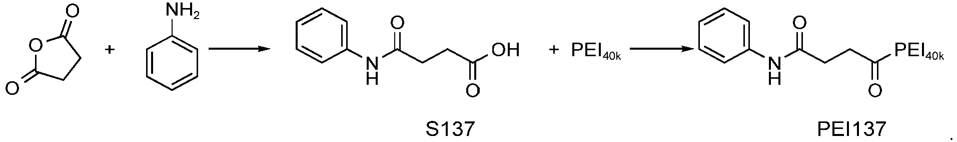
(1) One molar equivalent of aniline (10 g) was added to 100 mL of dichloromethane, 1.1 molar equivalents of succinic anhydride (11.8 g) and 2 equivalents of triethylamine (21.7 g) were added, and the mixture was stirred at room temperature for 4 hours, then concentrated to dry by rotary evaporation, and subjected to column chromatography to obtain pure Intermediate S137 (12 g) to be used directly in the next step with a yield of 62%;
   ¹H NMR (500 MHz, [D6] DMSO): δ=12.20 (br, 1H), 9.94 (br, 1H), 7.57 (d,J=7.6 Hz, 2H), 7.28 (t,J=7.9 Hz, 2H), 7.01 (t,J=7.4 Hz, 1H), 2.56-2.50 (m, 4H) ppm.
(2) To a round bottom flask, linear polyethyleneimine (0.8 g) having a weight average molecular mass of 40K was added, 40 mL of water was added, 0.2 molar equivalents of Intermediate S137 (0.386 g) and 3 molar equivalents of triethylamine (3.03 g) were added, and subsequently 40 mL of MeOH was added; after being stirred for 10 minutes, the mixture was added with 0.2 molar equivalents of 4-(4,6-dimethoxytriazin-2-yl)-4-methylmorpholinium hydrochloride (0.55 g), and stirred at room temperature overnight; then, MeOH was removed in vacuum, and water (200 mL) and subsequently 3M HCl solution (50 mL) were added; the residue was purified using a dialysis cassette in 100 mM HCl bath to obtain PEI 137(0.6 g), which had a yield of 50.6% and a grafting rate of 20%;
   ¹H NMR (500 MHz, D₂O): δ=7.29-7.11 (m, 5H), 3.89-3.23 (m, 20H), 2.68 (m, 4H).

### Example 2

A modified linear polyethyleneimine 101 (PEI 101) is provided, and a preparation method of PEI 101 includes the following steps: 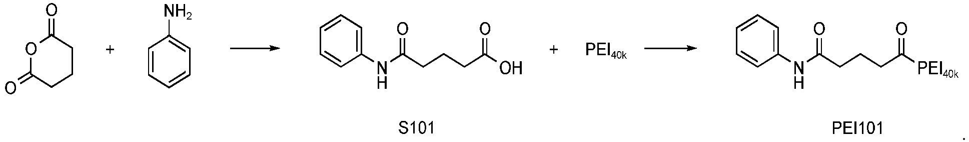
(1) Intermediate S101 was prepared with reference to step (1) of Example 1 with a yield of 78%; ¹H NMR (500 MHz, [D6] DMSO): δ=9.86 (s, 1H, NHAr), 7.58 (d, 2H), 7.3 (t, 2H), 6.98 (dd, 1H), 2.23 (m, 4H), 1.81 (m, 2H) ppm.
(2) PEI 101 was prepared with reference to step (2) of Example 1 with a yield of 65% and a grafting rate of 10%;
   ¹H NMR (500 MHz, D₂O): δ=7.27-7.06 (m, 5H), 3.87-3.11 (m, 40H), 2.44-2.26 (m, 4H), 1.83-1.75 (m, 2H) ppm.

### Example 3

A modified linear polyethyleneimine 105 (PEI 105) is provided, and a preparation method of PEI 105 includes the following steps: 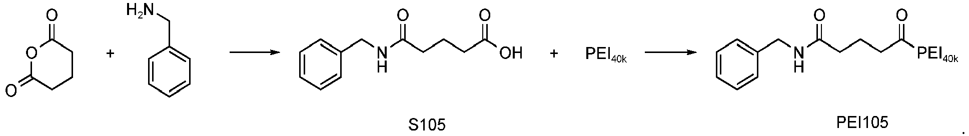
(1) Intermediate S105 was prepared with reference to step (1) of Example 1 with a yield of 63%; ¹H NMR (500 MHz, [D6] DMSO): δ=8.34 (s, 1H), 7.38-7.18 (m, 5H), 4.25 (d, 2H), 2.22-2.08 (m, 4H), 1.81-1.62 (m, 2H) ppm.
(2) PEI 105 was prepared with reference to step (2) of Example 1 with a yield of 58% and a grafting rate of 17%;
   ¹H NMR (500 MHz, D₂O): δ=7.24-7.12 (m, 5H), 4.30 (d, 2H), 3.87-3.11 (m, 23H), 2.42-2.18 (m, 4H), 1.80-1.68 (m, 2H) ppm.

### Example 4

A modified linear polyethyleneimine 107 (PEI 107) is provided, and a preparation method of PEI 107 includes the following steps: 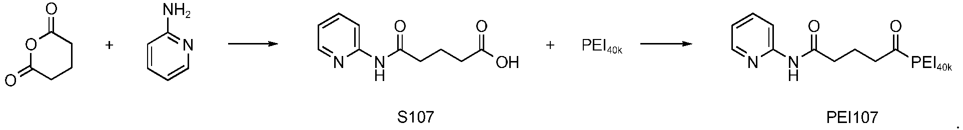
(1) Intermediate S107 was prepared with reference to step (1) of Example 1 with a yield of 63%; ¹H NMR (500 MHz, [D6] DMSO): δ=10.2 (s,1H), 8.29 (m, 1H), 8.09 (m, 1H), 7.76 (m, 1H), 7.27 (m, 1H), 2.30-2.01 (m, 4H), 1.85-1.76 (m, 2H) ppm.
(2) PEI 107 was prepared with reference to step (2) of Example 1 with a yield of 58% and a grafting rate of 15%;
   ¹H NMR (500 MHz, D₂O): δ=8.28-8.06 (m, 2H), 7.67-7.36 (m, 2H), 3.89-3.18 (m, 27H), 2.39-2.18 (m, 4H), 1.89-1.67 (m, 2H) ppm.

### Example 5

A modified linear polyethyleneimine 129 (PEI 129) is provided, and a preparation method of PEI 129 includes the following steps: 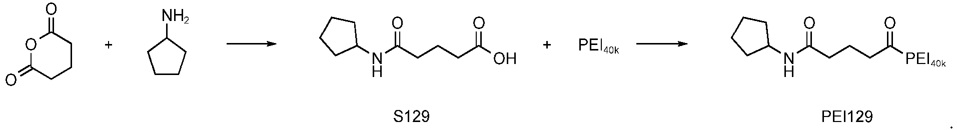
(1) Intermediate S129 was prepared with reference to step (1) of Example 1 with a yield of 43%; ¹H NMR (500 MHz, [D6] DMSO): δ=11.8 (s, 1H), 7.70-7.69 (d, 1H), 3.96-3.91 (m, 1H), 2.17-2.13 (m, 2H), 2.04-2.00 (m, 2H), 1.78-1.52 (m, 6 H), 1.49-1.44 (m, 2H), 1.34-1.27 (m, 2H) ppm.
(2) PEI 129 was prepared with reference to step (2) of Example 1 with a yield of 67% and a grafting rate of 20%;
   ¹H NMR (500 MHz, D₂O): δ=3.94-3.20 (m, 21H), 2.27-2.20 (m, 2H), 2.13-1.98 (m, 2H), 1.92-1.78 (m, 6H), 1.53-1.46 (m, 2H), 1.31-1.25 (m, 2H) ppm.

### Example 6

A modified linear polyethyleneimine 160 (PEI 160) is provided, and a preparation method of PEI 160 includes the following steps: 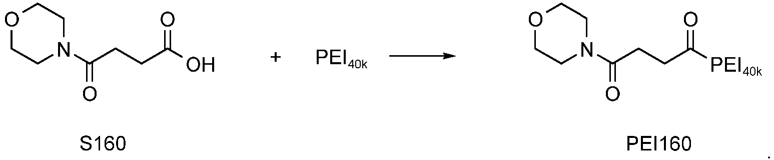
PEI 160 was prepared with reference to step (2) of Example 1 with a yield of 63% and a grafting rate of 26%;
¹H NMR (500 MHz, D₂O): δ=3.90-3.18 (m, 24H), 2.63 (m, 4H) ppm.

### Example 7

A modified linear polyethyleneimine 162 (PEI 162) is provided, and a preparation method of PEI 162 includes the following steps: 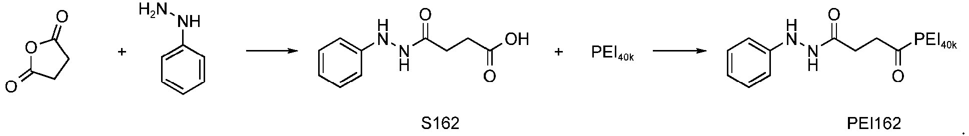
(1) Intermediate S162 was prepared with reference to step (1) of Example 1 with a yield of 86%; ¹H NMR (500 MHz, [D6] DMSO): δ=12.03 (s, 1H), 9.60 (d, 1H), 7.64 (d, 1H), 7.10 (d, 2H), 6.70-6.67 (m, 3H), 2.47 (t, 2H), 2.39 (t, 2H) ppm.
(2) PEI 162 was prepared with reference to step (2) of Example 1 with a yield of 70% and a grafting rate of 9%;
   ¹H NMR (500 MHz, D₂O): δ=7.15 (d, 2H), 6.80 (m, 3H), 3.90-3.2 (m, 44H), 2.60 (m, 4H) ppm.

### Example 8

A modified linear polyethyleneimine 164 (PEI 164) is provided, and a preparation method of PEI 164 includes the following steps: 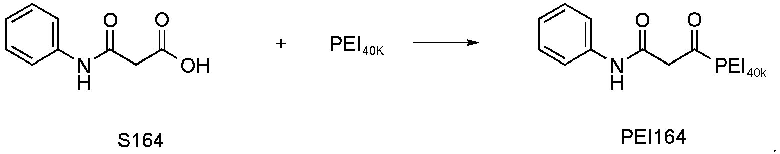
PEI 164 was prepared with reference to step (2) of Example 1 with a yield of 63% and a grafting rate of 17%;
¹H NMR (500 MHz, D₂O): δ=7.38-7.25 (m, 5H), 4.00-3.21 (m, 24H).

### Example 9

A modified linear polyethyleneimine 168 (PEI 168) is provided, and a preparation method of PEI 168 includes the following steps: 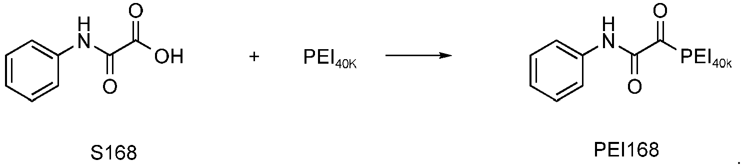
PEI 168 was prepared with reference to step (2) of Example 1 with a yield of 63% and a grafting rate of 10%;
¹H NMR (500 MHz, D₂O): δ=7.37-7.20 (m, 5H), 3.87-3.24 (m, 40H).

### Example 10

A modified linear polyethyleneimine 184 (PEI 184) is provided, and a preparation method of PEI 184 includes the following steps: 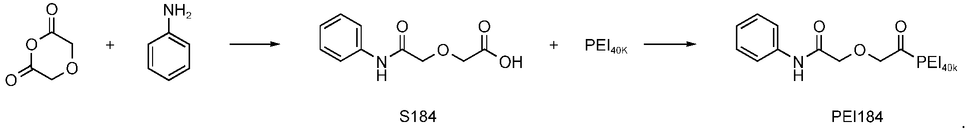
(1) Intermediate S184 was prepared with reference to step (1) of Example 1 with a yield of 57%; ¹H NMR (500 MHz, [D6] DMSO): δ=9.84 (s, 1H), 7.61 (d, 1H), 7.28 (d, 2H), 7.03 (d, 2H), 4.18 (s, 2H), 4.14 (s, 2H) ppm.
(2) PEI 184 was prepared with reference to step (2) of Example 1 with a yield of 50% and a grafting rate of 10%;
   ¹H NMR (500 MHz, D₂O): δ=7.35-7.18 (m, 5H), 4.41-3.20 (m, 45H) ppm.

Examples 1-10 provide preparation methods for different modified linear polyethyleneimines, and the modified linear polyethyleneimines with similar structures can be prepared according to related methods.

### Comparative Example 1

A modified linear polyethyleneimine 191 (PEI 191) is provided, and a preparation method of PEI 191 includes the following steps: 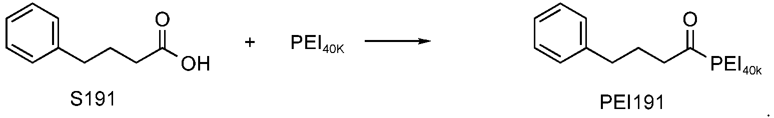
PEI 191 was prepared with reference to step (2) of Example 1 with a yield of 75% and a grafting rate of 20%;
¹H NMR (500 MHz, D₂O): δ=7.40-7.16 (m, 5H), 3.85-3.28 (m, 20H), 2.74(t, 2H), 2.47(t, 2H), 1.98 (m, 2H) ppm.

### Performance test

### Cell transfection assay

### (1) Cell culture

HEK-293F suspension cells (purchased from Wanjia Standard Substance) were grown in OPM-293CD-05 serum-free culture medium (OPM) at 37°C in an air atmosphere containing 5% CO₂.

### (2) Transfection assay of HEK-293F suspension cells (24-well plate)

On the day before transfection, HEK-293F suspension cells in good conditions were inoculated in 1 mL of complete medium by 2E⁵ cells per well (24-well plate), and cultured at 37°C in an air atmosphere containing 5% CO₂. On the day of transfection, 1 µg of MCS-EGFP plasmid (purchased from Weizhen Biotechnology) was added to 20 µL of OPM-293CD-05 serum-free culture medium (OPM), mixed via vortex, and incubated for 5 minutes at room temperature to obtain Suspension A (plasmid diluent); then, 2 µL of modified linear polyethyleneimine (1 g/L) was added to 20 µL of OPM-293CD-05 serum-free culture medium (OPM), mixed via vortex, and incubated for 5 minutes at room temperature to obtain Suspension B (modified linear polyethyleneimine diluent); Suspension B was added in Suspension A, mixed gently via vortex, and allowed to stand for 15 min at room temperature to form a plasmid-PEI complex for later use.

The plasmid-PEI complex was added by 40 µL into cells cultured in the 24-well plate, and the plate was cultured at 37°C in an air atmosphere containing 5% CO₂; a fluorescence microscope was used to collect images, then the cells were collected and subjected to counting and cell viability assay, and a flow cytometer (Manufacturer: Beckman; Model: A00-1-1102) was used to determine the positive rate of green fluorescent protein (GFP).

Polymers PEI 101, PEI 102, PEI 105, PEI 106, PEI 107, PEI 129, PEI 134, PEI 137, PEI 138, PEI 139, PEI 140, PEI 141, PEI 145, PEI 148, PEI 158, PEI 160, PEI 162, PEI 164, PEI 170, PEI 172, PEI 175, PEI 177, PEI 181, PEI 183, PEI 184, PEI 185, PEI 186, PEI 188, PEI 189, PEI 190 provided by the present application and PEI 191 and PEI MAX (linear polyethyleneimine, with a weight average molecular mass of 40K) provided by Comparative Example 1 were tested according to the above testing methods, and the test results are shown in FIG. 1. As can be seen from FIG. 1, compared with the bare cationic polymer without modification (PEI MAX) or the cationic polymer (PEI 191) modified by only one amide bond, the modified linear polyethyleneimine provided by the present application has obviously improved transfection efficiency; because two amide bonds modified on the linear PEI can both form hydrogen bonds with heteroatoms on nucleic acids, the nucleic acid molecules are further compressed, and the resulting firmer complexes can be more easily taken into cells through endocytosis.

The applicant has stated that the detailed process flow of the present application is illustrated by the above examples, but the present application is not limited to the above detailed process flow, which means that the present application does not necessarily rely on the above detailed process flow to be practiced. It should be apparent to those skilled in the art that any modification to the present application, equivalent substitution of various raw materials and addition of auxiliary ingredients to the products of the present application, selection of specific methods, etc., shall fall within the protection scope and the disclosure scope of the present application.

## Claims

1. A modified cationic polymer, which has a structure shown by Formula I or Formula II: 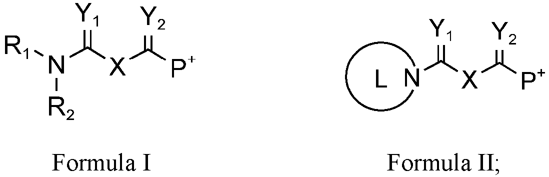
wherein R₁ and R₂ are each independently selected from any one of hydrogen, substituted or unsubstituted amino, substituted or unsubstituted C1-C20 linear or branched alkyl, substituted or unsubstituted C3-C30 cycloalkyl, C2-C30 heterocycloalkyl, substituted or unsubstituted C1-C20 alkoxy, substituted or unsubstituted C6-C30 aryl, and substituted or unsubstituted C3-C30 heteroaryl; one or at least two nonadjacent -CH₂- in the C1-C20 linear or branched alkyl can each independently be substituted with -O-;
X is selected from any one of a single bond, substituted or unsubstituted C1-C10 linear or branched alkylene, C2-C10 linear or branched alkenylene, C2-C10 linear or branched alkynylene, C3-C10 cycloalkylene, C3-C10 heterocycloalkylene, and C3-C10 unsaturated cycloalkylene; in the C1-C10 linear or branched alkylene, one or at least two nonadjacent -CH₂- can each independently be substituted with -O- or -S-, and one or at least two nonadjacent -CH- can each independently be substituted with -N-;
Y₁ and Y₂ are each independently selected from O or S;
the ring L is selected from any one of a substituted or unsubstituted C3-C10 N-containing heteroalicyclic ring, and a substituted or unsubstituted C3-C12 N-containing heteroaromatic ring;
P⁺ represents a cationic polymer; and the cationic polymer comprises any one of cationic polymers containing primary amine and/or secondary amine;
substituents for substitution in R₁, R₂, X, and the ring L are each independently selected from at least one of unsubstituted or Z₁-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino;
M₁ is selected from any one of C1-C10 linear or branched alkyl; and
Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

2. The modified cationic polymer according to claim 1, wherein R₁ and R₂ are each independently selected from any one of hydrogen, substituted or unsubstituted amino, substituted or unsubstituted C1-C10 linear or branched alkyl, substituted or unsubstituted C3-C10 cycloalkyl, C2-C10 heterocycloalkyl, substituted or unsubstituted C1-C10 alkoxy, substituted or unsubstituted C6-C20 aryl, and substituted or unsubstituted C3-C20 heteroaryl; one or at least two nonadjacent -CH₂- in the C1-C10 linear or branched alkyl can each independently be substituted with -O-;
a substituent for substitution is selected from at least one of unsubstituted or Z₁-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino;
M₁ is selected from any one of C1-C10 linear or branched alkyl; and
Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

3. The modified cationic polymer according to claim 1 or 2, wherein one of R₁ and R₂ is hydrogen, and the other one is selected from any one of substituted or unsubstituted amino, substituted or unsubstituted C1-C10 linear or branched alkyl, C3-C10 cycloalkyl, substituted or unsubstituted C6-C20 aryl, and substituted or unsubstituted C3-C20 heteroaryl; one or at least two nonadjacent -CH2- in the C1-C10 linear or branched alkyl can each independently be substituted with -O-;
a substituent for substitution is selected from at least one of unsubstituted or Z₁-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino;
M₁ is selected from any one of C1-C10 linear or branched alkyl;
Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl;
preferably, one of R₁ and R₂ is hydrogen, and the other one is selected from any one of substituted or unsubstituted C1-C5 linear alkyl, substituted or unsubstituted amino, CH₃O(CH₂)₂O(CH₂)₂-, CH₃O(CH₂)₂O(CH₂)₂O(CH₂)₂-, and the following groups in substituted or unsubstituted form: 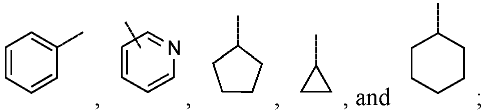
wherein the dotted line represents a linkage site for the group;
a substituent for substitution is selected from at least one of unsubstituted or Zi-substituted C3-C10 cycloalkyl, unsubstituted or Z₂-substituted C1-C10 linear or branched alkyl, halogen, unsubstituted or Z₃-substituted C6-C12 aryl, C3-C12 heteroaryl, C1-C10 alkoxy, C3-C10 heterocycloalkyl, hydroxyl, M₁-OC(=O)-, and unsubstituted or Z₄-substituted amino;
M₁ is selected from any one of C1-C10 linear or branched alkyl; and
Z₁ and Z₄ are each independently selected from at least one of C1-C10 linear or branched alkyl; Z₂ is selected from at least one of C6-C12 aryl, and halogen; Z₃ is selected from at least one of halogen, hydroxyl, and C1-C10 linear or branched alkyl.

4. The modified cationic polymer according to any one of claims 1-3, wherein X is selected from any one of a single bond, C1-C5 linear or branched alkylene, C2-C5 linear or branched alkenylene, C2-C5 linear or branched alkynylene, C3-C6 cycloalkylene, C3-C6 heterocycloalkylene, and C3-C6 unsaturated cycloalkylene; in the C1-C5 linear or branched alkylene, one or at least two nonadjacent -CH₂- can each independently be substituted with -O- or -S-, and one or at least two nonadjacent -CH- can each independently be substituted with -N-;
preferably, X is selected from any one of ethylidene, propylidene, butylidene, vinylidene or propenylidene.

5. The modified cationic polymer according to any one of claims 1-4, wherein the ring L is selected from any one of a C3-C6 N-containing heteroalicyclic ring and a C3-C7 N-containing heteroaromatic ring;
preferably, the ring L is selected from any one of the following groups: 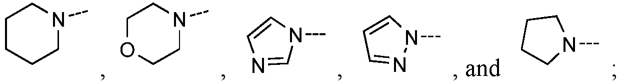
wherein the dotted line represents a linkage site for the group.

6. The modified cationic polymer according to any one of claims 1-5, wherein the cationic polymers containing primary amine and/or secondary amine comprise any one of branched polyethyleneimine, linear polyethyleneimine, polyallylamine, polylysine or polyvinylamine, and further preferably linear polyethyleneimine.

7. The modified cationic polymer according to any one of claims 1-6, wherein the cationic polymers containing primary amine and/or secondary amine have a weight average molecular mass of 5000-50000, and further preferably 15000-40000;
preferably, the modified cationic polymer has a grafting rate of 8-36%.

8. The modified cationic polymer according to any one of claims 1-7, wherein the modified cationic polymer is modified linear polyethyleneimine having any one of the following structures: 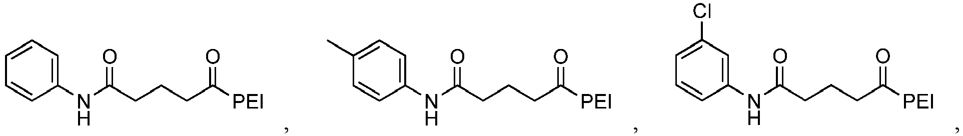 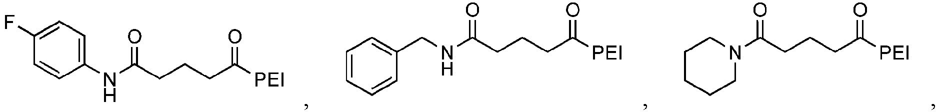 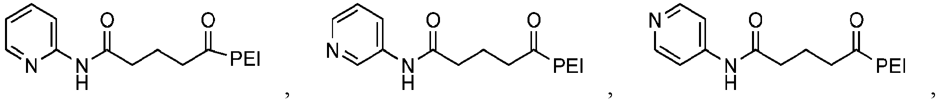 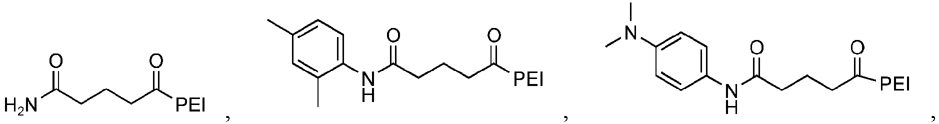 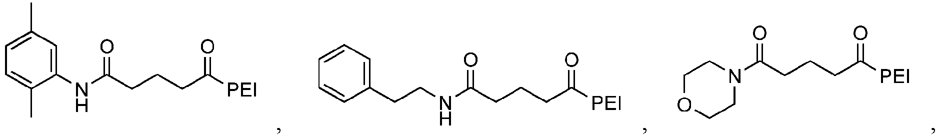 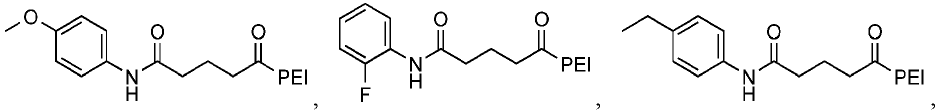 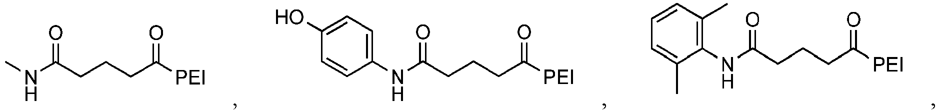 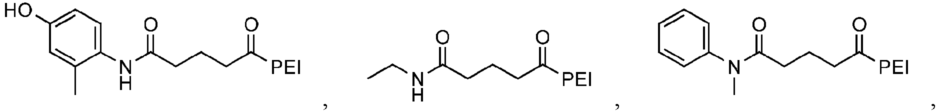 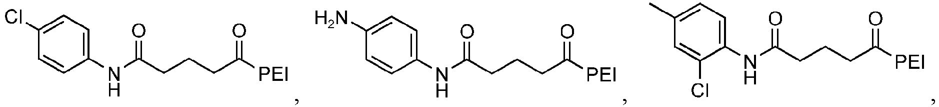 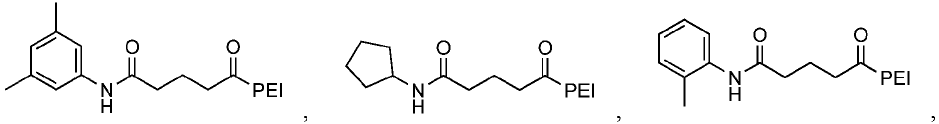 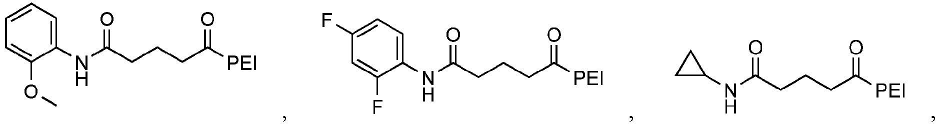 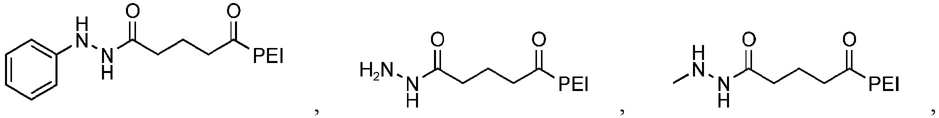 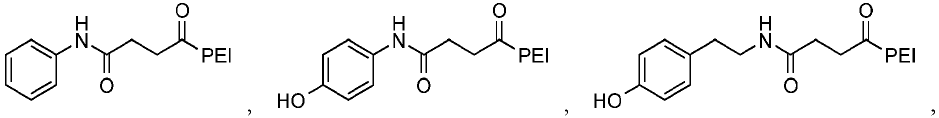 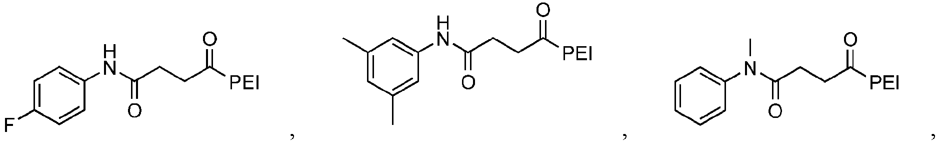 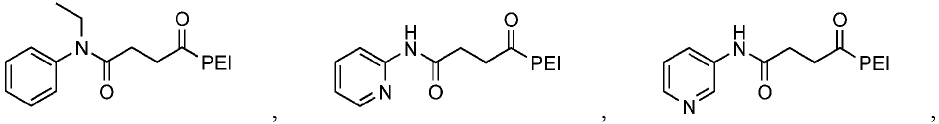 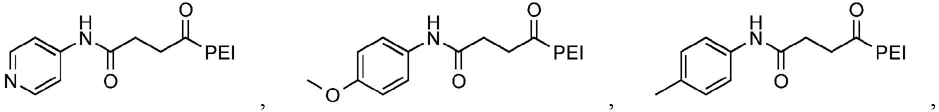 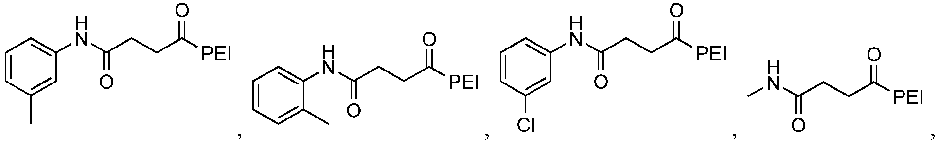 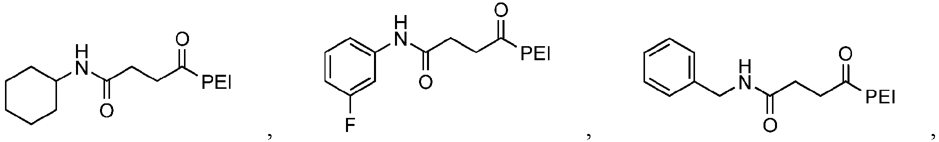 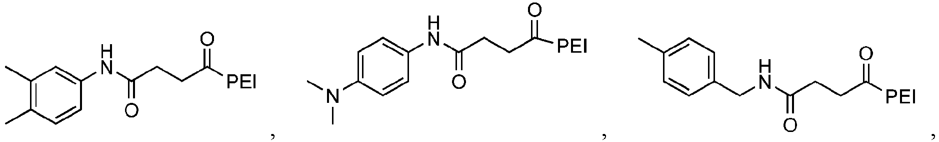 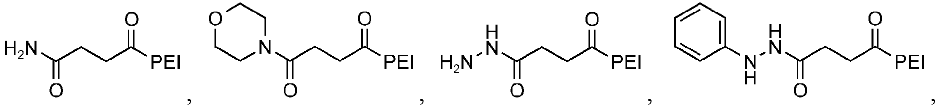 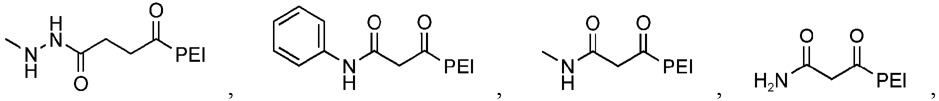 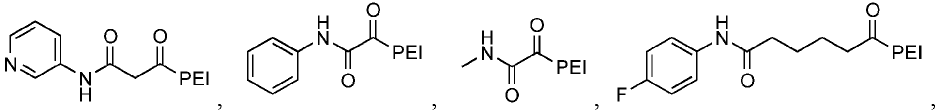 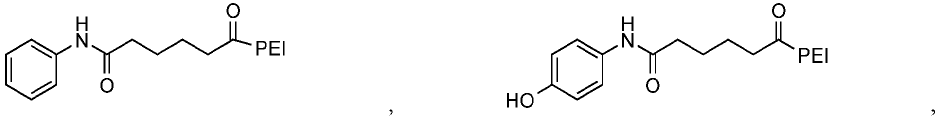 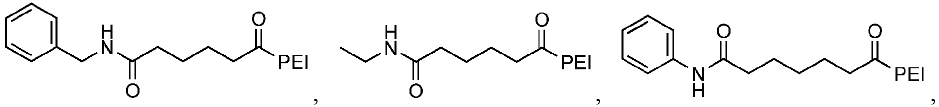 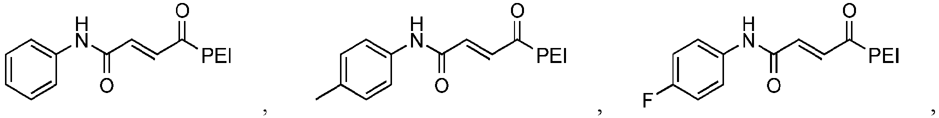 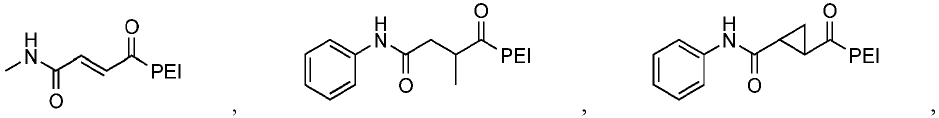 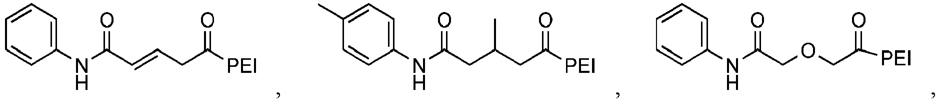 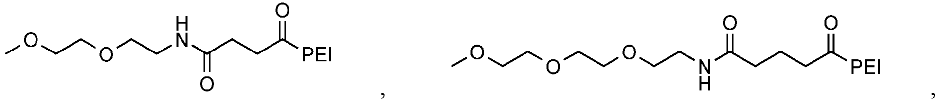 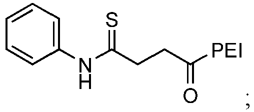 preferably, the modified cationic polymer comprises any one of the following modified linear polyethyleneimines 101-190:
| No. | Structure | Weight average molecular mass of linear polyethyleneimine | Grafting rate |
|---|---|---|---|
| 101 | 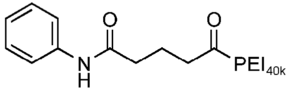 | 40K | 10% |
| 102 | 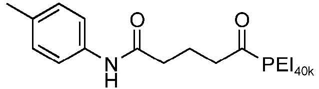 | 40K | 20% |
| 103 | 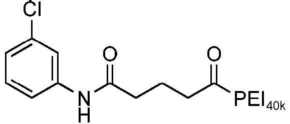 | 40K | 13% |
| 104 | 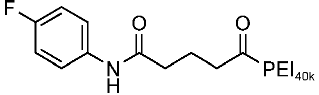 | 40K | 18% |
| 105 | 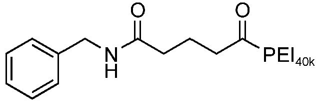 | 40K | 17% |
| 106 | 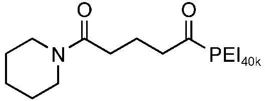 | 40K | 20% |
| 107 | 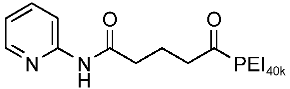 | 40K | 15% |
| 108 | 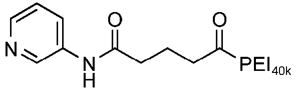 | 40K | 8% |
| 109 | 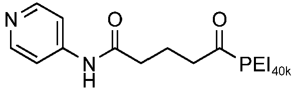 | 40K | 10% |
| 110 | 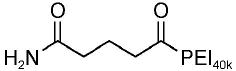 | 40K | 20% |
| 111 | 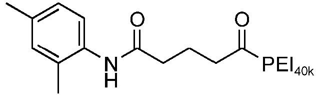 | 40K | 20% |
| 112 | 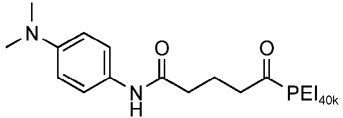 | 40K | 25% |
| 113 | 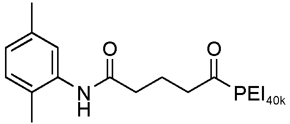 | 40K | 21% |
| 114 | 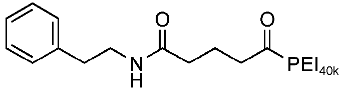 | 40K | 15% |
| 115 | 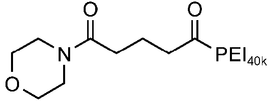 | 40K | 20% |
| 116 | 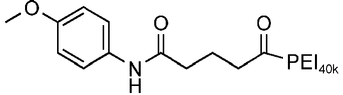 | 40K | 20% |
| 117 | 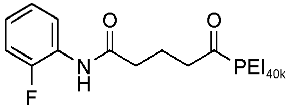 | 40K | 15% |
| 118 | 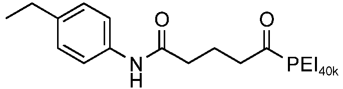 | 40K | 18% |
| 119 | 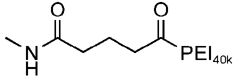 | 40K | 16% |
| 120 | 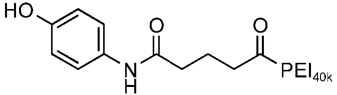 | 40K | 18% |
| 121 | 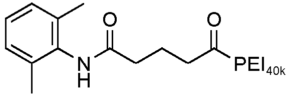 | 40K | 10% |
| 122 | 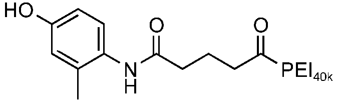 | 40K | 15% |
| 123 | 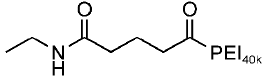 | 40K | 18% |
| 124 | 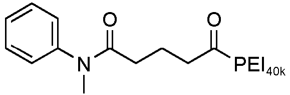 | 40K | 20% |
| 125 | 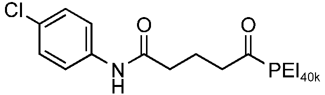 | 40K | 18% |
| 126 | 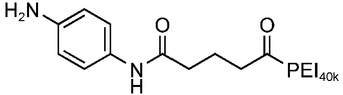 | 40K | 25% |
| 127 | 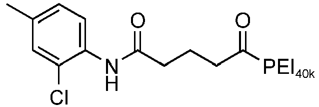 | 40K | 26% |
| 128 | 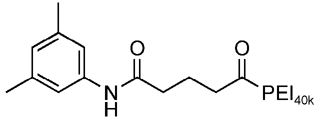 | 40K | 30% |
| 129 | 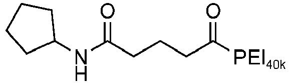 | 40K | 20% |
| 130 | 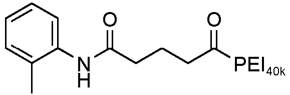 | 40K | 30% |
| 131 | 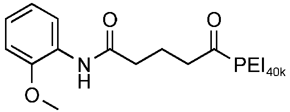 | 40K | 20% |
| 132 | 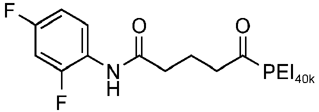 | 40K | 20% |
| 133 | 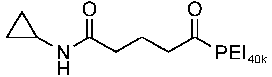 | 40K | 10% |
| 134 | 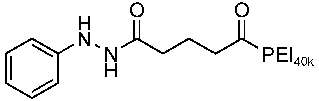 | 40K | 15% |
| 135 | 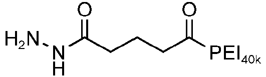 | 40K | 20% |
| 136 | 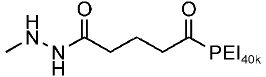 | 40K | 20% |
| 137 | 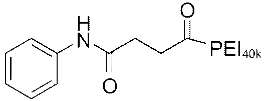 | 40K | 20% |
| 138 | 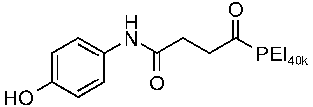 | 40K | 35% |
| 139 | 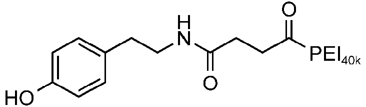 | 40K | 20% |
| 140 | 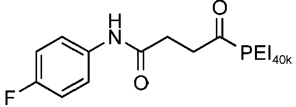 | 40K | 25% |
| 141 | 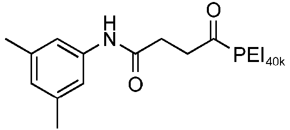 | 40K | 25% |
| 142 | 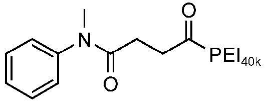 | 40K | 10% |
| 143 | 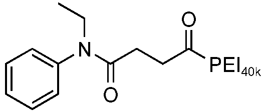 | 40K | 15% |
| 144 | 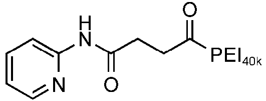 | 40K | 25% |
| 145 | 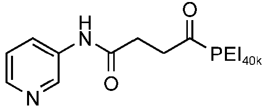 | 40K | 24% |
| 146 | 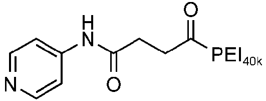 | 40K | 20% |
| 147 | 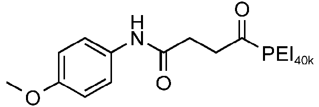 | 40K | 18% |
| 148 | 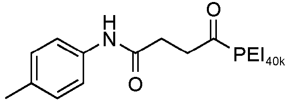 | 40K | 28% |
| 149 | 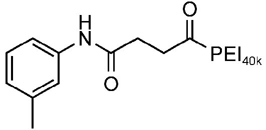 | 40K | 35% |
| 150 | 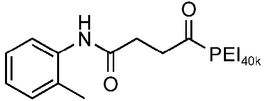 | 40K | 15% |
| 151 | 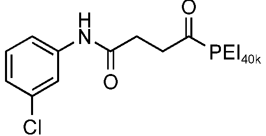 | 40K | 20% |
| 152 | 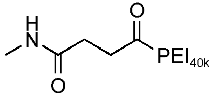 | 40K | 10% |
| 153 | 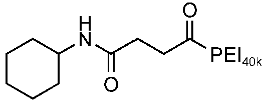 | 40K | 17% |
| 154 | 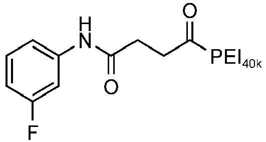 | 40K | 26% |
| 155 | 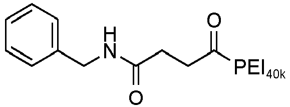 | 40K | 28% |
| 156 | 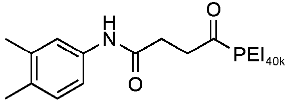 | 40K | 20% |
| 157 | 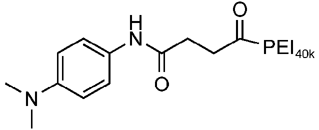 | 40K | 15% |
| 158 | 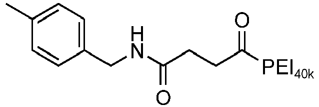 | 40K | 24% |
| 159 | 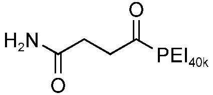 | 40K | 20% |
| 160 | 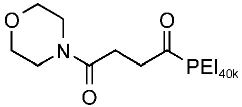 | 40K | 26% |
| 161 | 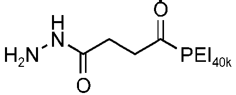 | 40K | 15% |
| 162 | 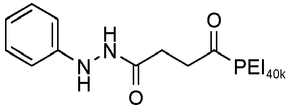 | 40K | 9% |
| 163 | 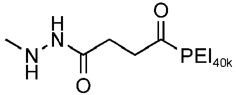 | 40K | 13% |
| 164 | 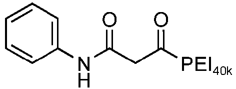 | 40K | 17% |
| 165 | 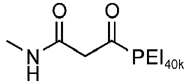 | 40K | 16% |
| 166 | 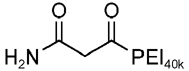 | 40K | 15% |
| 167 | 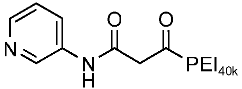 | 40K | 20% |
| 168 | 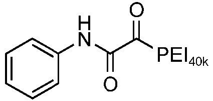 | 40K | 10% |
| 169 | 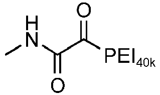 | 40K | 25% |
| 170 | 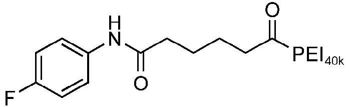 | 40K | 26% |
| 171 | 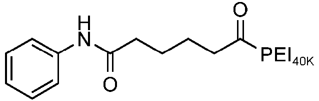 | 40K | 15% |
| 172 | 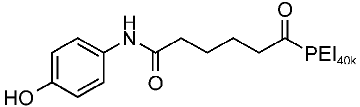 | 40K | 17% |
| 173 | 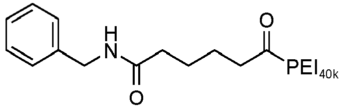 | 40K | 22% |
| 174 | 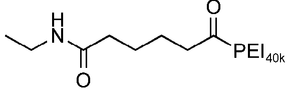 | 40K | 16% |
| 175 | 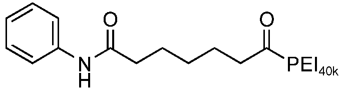 | 40K | 20% |
| 176 | 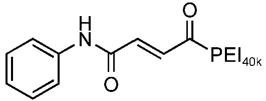 | 40K | 20% |
| 177 | 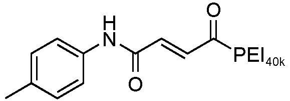 | 40K | 17% |
| 178 | 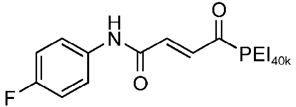 | 40K | 24% |
| 179 | 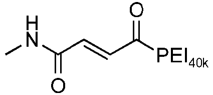 | 40K | 12% |
| 180 | 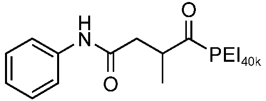 | 40K | 36% |
| 181 | 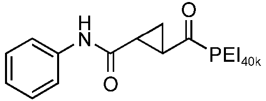 | 40K | 13% |
| 182 | 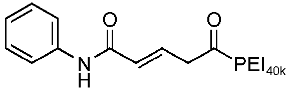 | 40K | 15% |
| 183 | 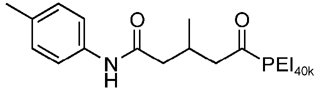 | 40K | 20% |
| 184 | 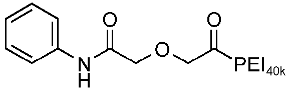 | 40K | 10% |
| 185 | 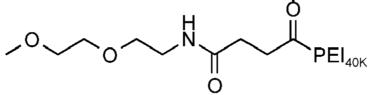 | 40K | 20% |
| 186 | 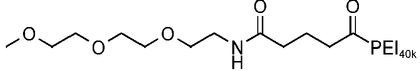 | 40K | 16% |
| 187 | 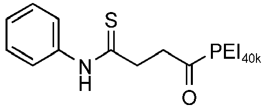 | 40K | 20% |
| 188 | 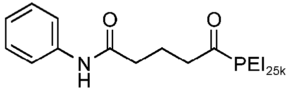 | 25K | 10% |
| 189 | 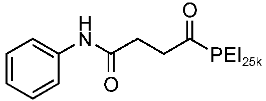 | 25K | 20% |
| 190 | 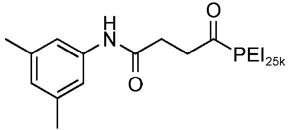 | 25K | 25%; |
preferably, the modified cationic polymer has a structure shown by Formula I-1 or Formula II-1: 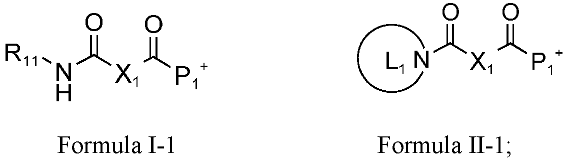
wherein R₁₁ is selected from any one of phenyl-substituted C1-C5 linear alkyl, phenyl-substituted amino, substituted or unsubstituted C6-C12 aryl, substituted or unsubstituted C3-C12 heteroaryl, and C3-C10 cycloalkyl;
X₁ is selected from any one of a single bond and C1-C4 linear alkylene; one or at least two nonadjacent -CH₂- in the C1-C4 linear alkylene can each independently be substituted with -O-;
the ring L₁ is selected from any one of a substituted or unsubstituted C3-C10 N-containing heteroalicyclic ring, and a substituted or unsubstituted C3-C12 N-containing heteroaromatic ring;
P₁⁺ represents linear polyethyleneimine;
a substituent for substitution is selected from at least one of halogen, hydroxyl, and C1-C10 linear or branched alkyl;
preferably, the R₁₁ is selected from any one of the following groups: 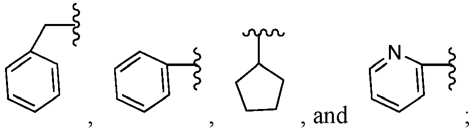
wherein 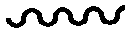 represents a linkage site for the group;
preferably, the R₁₁ is selected from any one of C6-C12 aryl;
preferably, the X₁ is selected from any one of C1-C4 linear alkylene;
preferably, the modified cationic polymer comprises any one of the following modified linear polyethyleneimines:
| Structure | Weight average molecular mass of linear polyethyleneimine | Grafting rate |
|---|---|---|
| 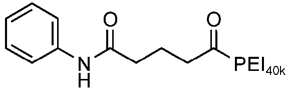 | 40K | 10% |
| 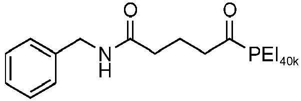 | 40K | 17% |
| 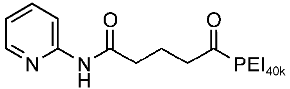 | 40K | 15% |
| 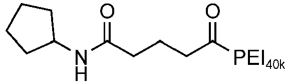 | 40K | 20% |
| 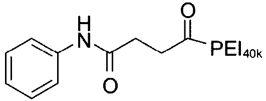 | 40K | 20% |
| 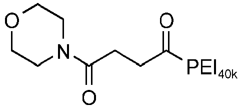 | 40K | 26% |
| 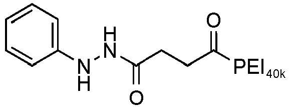 | 40K | 9% |
| 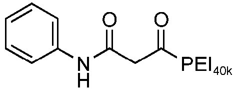 | 40K | 17% |
| 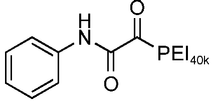 | 40K | 10% |
| 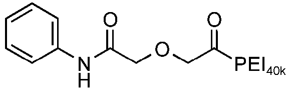 | 40K | 10%. |

9. A tautomer, mesomer, racemate, enantiomer, diastereomer or acceptable salt of the modified cationic polymer according to any one of claims 1-8.

10. A transfection reagent, comprising any one or a combination of at least two of the modified cationic polymer according to any one of claims 1-8, or the tautomer, mesomer, racemate, enantiomer, diastereomer or acceptable salt of the modified cationic polymer according to claim 9.
